# EUROPÄISCHE PATENTSCHRIFT

(11) **EP 3 242 870 B1**
(45) Veröffentlichungstag und Bekanntmachung des Hinweises auf die Patenterteilung: **13.03.2019**
(21) Anmeldenummer: 16700181.7
(22) Anmeldetag: 08.01.2016
(51) Int. Cl.: C07D 307/08, C07C 29/149, C07D 307/33, C07C 51/573, C12P 7/18, C12P 17/04

(54) **VERFAHREN ZUR HERSTELLUNG VON TETRAHYDROFURAN, 1,4-BUTANDIOL ODER GAMMA-BUTYROLACTON**
METHOD FOR THE PRODUCTION OF TETRAHYDROFURANE, 1,4-BUTANEDIOL OR GAMMA-BUTYROLACTONE
PROCÉDÉ DE FABRICATION DU TÉTRAHYDROFURANNE, DU 1,4-BUTANEDIOL OU DE LA GAMMA-BUTYROLACTONE

(30) Priorität: 09.01.2015 EP 15150615
(43) Veröffentlichungstag der Anmeldung: 15.11.2017
(73) Patentinhaber: BASF SE, 67056 Ludwigshafen (DE)
(72) Erfinder: DUEFERT, Alexander, 67063 Ludwigshafen (DE); PINKOS, Rolf, 67098 Bad Duerkheim (DE); WEISSKER, Wolf-Steffen, 67245 Lambsheim (DE)
(74) Vertreter: BASF IP Association
(86) Internationale Anmeldenummer: PCT/EP2016/050245
(87) Internationale Veröffentlichungsnummer: WO 2016/110556

(56) Entgegenhaltungen:
- WO-A1-2010/092155
- DE-B- 1 141 282
- JP-A- 2003 113 171

## Beschreibung

Die Erfindung betrifft ein Verfahren zur Herstellung von Tetrahydrofuran und/oder 1,4-Butandiol und/oder gamma-Butyrolacton, insbesondere ein Verfahren zur Herstellung von Tetrahydrofuran (THF) aus Bernsteinsäure, die durch Umsetzung von Biomasse erhalten wurde, durch Umwandlung der Bernsteinsäure in Bernsteinsäureanhydrid und Hydrierung des Bernsteinsäureanhydrids, unter Abtrennung störender Nebenkomponenten.

Die Herstellung von THF durch Hydrierung von Carbonsäurederivaten wie Maleinsäureanhydrid, Maleinsäure, Maleinsäureester, Bernsteinsäureanhydrid, Bernsteinsäure sowie Bernsteinsäurediester ist an sich bekannt. So beschreibt die DE 100 61 556 A1 die Hydrierung von Dicarbonsäuren sowie deren Derivaten an Cu-Katalysatoren in der Gasphase. Schwerpunkt ist die Hydrierung von durch Gasphasenoxidation von z.B. Butan hergestelltem Maleinsäureanhydrid. Ähnliches beschreibt die WO 2003 006 446, wobei hier der Schwerpunkt auf der Hydrierung von Diestern liegt. In keinem der Dokumente ist erwähnt, wie man ausgehend von fermentativ hergestellter Bernsteinsäure THF herstellt.

In EP 2 476 674 A2 ist beschrieben, wie man cyclische Verbindungen (Lactone und Ether) ausgehend von C₄-C₆-Carbonsäuren oder deren Estern herstellt. Ausdrücklich erwähnt ist die Verwendung von Biomasse-basierten Säuren bzw. daraus hergestellten Estern. Bevorzugt wird ein Katalysator verwendet, der neutral ist, damit keine durch Dehydratisierung entstehenden Nebenprodukte generiert werden. Die Verwendung von Bernsteinsäureanhydrid ist nicht erwähnt. Ebensowenig wird gelehrt, wie man vorhandene Verunreinigungen in den Ausgangsstoffen abtrennt bzw. deren Einfluss möglichst eingrenzt.

Die Herstellung von Bernsteinsäure aus Biomasse ist beispielsweise in WO 2010/092155 A1 beschrieben. Ferner beschreibt diese WO auch die Weiterverwendung von Bernsteinsäure oder ihrem Diester zur Gewinnung von THF, Butandiol und/oder gamma-Butyrolacton, wobei die Ester beispielsweise durch Reaktivdestillationsveresterung von Diammoniumsuccinat erhalten werden. Ansonsten werden Salze der Bernsteinsäure durch saure lonentauscher, die dann beispielsweise mit HCl regeneriert werden, in freie Bernsteinsäure umgewandelt. Die Reinigung dieser so erhaltenen Bernsteinsäure erfolgt dann durch Aufkonzentration und Kristallisation. In Beispiel 9 wird die Reinheit der Bernsteinsäure mit 99,8 % angegeben. Nicht beschrieben ist, welche Verunreinigungen enthalten sind und wie diese dann ggf. die Hydrierungen beeinflussen. Auch Bernsteinsäureanhydrid ist nicht erwähnt.

Die Herstellung von Bernsteinsäureanhydrid aus Bernsteinsäure ist an sich bekannt. In der DE-A-1 141 282 wird die Herstellung von Bernsteinsäureanhydrid durch Zuführung flüssiger Bernsteinsäure in eine Kolonne beschrieben, wobei Wasser über Kopf abdestilliert und das Anhydrid mit einer Reinheit von 95 - 97 % über Sumpf anfällt. Über die Verunreinigungen wird nichts ausgesagt. Gemäß FR 1 386 278 wird Bernsteinsäure destillativ entwässert, wobei das Anhydrid im Rückstand verbleibt. Gemäß Org. Lett. 2011, 13, 892 wird Bernsteinsäure in einem homogen-katalysierten Verfahren mit dem Katalysator als Schwersieder gebildet; die Aufreinigung erfolgt durch Fällung des Anhydrids und Filtration der Reaktionsmischung.

JP 2003 113 3171 A beschreibt die Reinigung von Bernsteinsäureanhydrid durch Destillation, wobei die Destillation der Rohbernsteinsäure zur Vermeidung von Verfärbungen des Produktes die Sumpftemperatur bei Unterdruck zwischen 125 und 200 °C liegt. Lediglich Dilactone werden als zu vermeidende Verunreinigungen beschrieben. Nicht erwähnt ist die Herstellung des Bernsteinsäureanhydrids durch aus fermentativen Verfahren gewonnener Bernsteinsäure. Des Weiteren wird eine Auswirkung auf nachfolgende Hydrierschritte nicht ausgeführt, nur eine Verfärbung von mit verunreinigtem Bernsteinsäureanhydrid hergestellten Polyestern wird erwähnt.

Im Gegensatz zu Produkten die durch klassische chemische Reaktionen hergestellt werden, liegt die Besonderheit von Produkten, die aus Biomasse erhalten werden, in der ungleich höheren Anzahl von Nebenkomponenten, die nachfolgende Anwendungen stören können, vor allem, wenn Polymere mit langen Kettenlängen entstehen sollen, bei denen monofunktionelle Gruppen den Aufbau von Ketten stören. Im hier vorliegenden Fall ist eine Hauptanwendung von THF die Herstellung von Polytetrahydrofuran. Ferner können Nebenkomponenten störend auf Katalysatoren bezüglich Selektivität und Ausbeute des Prozesses einwirken, vor allem aber die Lebensdauer der Katalysatoren negativ beeinflussen. Beispiele dieser störenden Nebenkomponenten, die bereits in Mengen unterhalb von 1 Gew.-ppm schädlich sein können, enthalten die Elemente N, P, S, As, Sb, Bi, Sn und Halogene wie Cl, Br und I.

N-enthaltende Verbindungen, vor allem, wenn sie basische Eigenschaften besitzen, können saure Zentren auf Katalysatoren belegen und damit gewünschte Eigenschaften zerstören. Insbesondere sind die Stickstoff-enthaltenden Verbindungen schädlich, wenn sie einen Hydrierschritt durchlaufen, da dabei viele von ihnen erst zu basisch wirkenden Verbindungen werden. Dies kann dann den Hydrierschritt oder nachfolgende Prozesse behindern. Hier ist wiederum die Polymerisation von THF negativ betroffen, da diese in Gegenwart sauer Katalysatoren durchgeführt wird. Insbesondere bei der Hydrierung von Bernsteinsäureanhydrid in Gegenwart von N-haltigen Verbindungen, insbesondere von Ammoniak oder solchen, die diesen freisetzen können, kann leicht Pyrrolidin entstehen, das den Hydrierprozess behindert und aufgrund seines Siedepunktes ähnlich wie THF von diesem nur schwer abtrennbar ist, und später die Polymerisation stört.

Verbindungen, die P, S, As, Sb, Bi, Sn oder Halogene wie Cl, Br und I enthalten, sind unerwünscht, da sie zum Teil nicht nur für die Umwelt giftig sind, sondern auch Hydrierkatalysatoren vergiften können. Viele dieser Verbindungen sind flüchtig, so dass sie destillative Reinigungsprozesse zum Teil überstehen bzw. in Gasphasenprozessen in den zu hydrierenden Strom gelangen können.

In fermentativen Prozessen zur Herstellung von Bernsteinsäure entsteht beispielsweise als Säure nicht nur Bernsteinsäure, sondern auch eine Reihe anderer Säuren wie Ameisensäure, Essigsäure, Propionsäure und Buttersäure. Diese sind aufgrund ihrer Säurestärke in der Lage, Katalysatoren zu schädigen.

Aufgabe der vorliegenden Erfindung ist die Bereitstellung eines Verfahrens zur Herstellung von Tetrahydrofuran und/oder 1,4-Butandiol und/oder gamma-Butyrolacton, vorzugsweise von Tetrahydrofuran (THF), aus fermentativ hergestellter Bernsteinsäure, das die Nachteile bekannter Verfahren vermeidet und die gewünschten Produkte in hoher Ausbeute und Reinheit liefert. Der in dem notwendigen Hydrierschritt eingesetzte Hydrierkatalysator soll eine lange Lebensdauer aufweisen. Zudem soll das Verfahren möglichst unaufwendig durchzuführen sein.

Die Aufgabe wird gelöst durch ein Verfahren zur Herstellung von Tetrahydrofuran und/oder 1,4-Butandiol und/oder gamma-Butyrolacton, umfassend die Schritte
a) fermentative Herstellung von Bernsteinsäure,
b) Überführung der Bernsteinsäure aus Schritt a) unter Wasserabspaltung und Wasserabtrennung in Bernsteinsäureanhydrid,
c) Überführung des Bernsteinsäureanhydrids aus Schritt b) in die Gasphase,
d) Entfernen von schwefelhaltigen Verbindungen aus dem Bernsteinsäureanhydrid durch Leiten des Bernsteinsäureanhydrids aus Schritt c) über ein festes Schutzbett (guard bed), das die schwefelhaltigen Verbindungen absorbiert,
e) Hydrieren des gasförmigen Bernsteinsäureanhydrids aus Schritt d) in Gegenwart von freiem Wasserstoff an einem metallhaltigen Katalysator zu Tetrahydrofuran und/oder 1,4-Butandiol und/oder gamma-Butyrolacton.

Es wurde erfindungsgemäß gefunden, dass bei der Herstellung von Tetrahydrofuran, 1,4-Butandiol und/oder gamma-Butyrolacton ausgehend von Bernsteinsäure die Hydrierung mit einer hohen Lebensdauer des Katalysators durchgeführt werden kann, wenn fermentativ hergestellte Bernsteinsäure über das Bernsteinsäureanhydrid aufgereinigt wird, und schwefelhaltige Verbindungen aus dem Bernsteinsäureanhydrid mithilfe eines festen Schutzbettes (guard bed) entfernt werden.

1,4-Butandiol kann später mit Dicarbonsäuren oder Diisocyanaten weiter umgesetzt werden.

Neben schwefelhaltigen Verbindungen werden vorzugsweise auch weitere störende Verbindungen, die P, As, Sb, Bi, Sn oder Halogene wie Cl, Br oder I enthalten, durch das Schutzbett ab- und/oder absorbiert, so dass sie nicht in den zu hydrierenden Strom gelangen, der mit dem Hydrierkatalysator kontaktiert wird.

### Schritt a)

Fermentation bezeichnet in der Biologie eine Form von enzymatischer Umwandlung organischer Stoffe. Die Fermentation wird bei vielen biotechnologischen Produktionsverfahren angewendet. Dies geschieht beispielsweise durch Zugabe der benötigten Enzyme oder durch Zugabe von Bakterien-, Pilz- oder sonstigen biologischen Zellkulturen, die die Fermentation im Rahmen ihres enzymkatalysierten Stoffwechsels ausführen.

Dabei enthält die Fermentationsbrühe vorzugsweise Enzyme, Bakterien, Pilze und/oder sonstige biologische Zellkulturen. Zudem enthält die Fermentationsbrühe Biomasse.

Unter Biomasse werden beispielsweise Stoffe verstanden, die entweder direkt in der Natur vorkommen wie Stärke, Zellulose oder Zucker, oder daraus abgeleitet sind wie Glycerin und durch Spaltung entstandene Zucker wie Glukose, Saccharose usw., sowie CO₂. In diesem Zusammenhang sei auf WO 2010/092155 A1 und die darin genannten Rohstoffquellen verwiesen. Die bevorzugte Herstellung der Bernsteinsäure verläuft über Fermentation.

Die benötigten Mikroorganismen können auf den Ausgangsstoffen bereits vorhanden sein. Bevorzugt werden jedoch in erfindungsgemäßen Fermentationsverfahren Reinzuchtzellkulturen zugegeben, um die Fermentation besser zu kontrollieren und unerwünschte Nebenprodukte auszuschließen. Daher ist die sterile Betriebsweise des Reaktors wichtig.

Das Haupteinsatzgebiet der Fermentation ist die Biotechnologie zur Herstellung unterschiedlichster Fermentationsprodukte, wie Bioethanol, Aminosäuren, organische Säuren, wie Milchsäure, Zitronensäure und Essigsäure, Enzyme, wie Phytase, Antibiotika und andere pharmazeutische Produkte, Biomonomere und Biopolymere.

Für eine detaillierte Beschreibung der Fermentation kann auf WO 2009/024294 und WO 2010/092155 verwiesen werden. Die erfindungsgemäßen Reaktoren können gerührte Fermentierer sowie auch Blasensäulen ersetzen. Fermentationsverfahren sind allgemein in Chmiel; Bioprozesstechnik: Einführung in die Bioverfahrenstechnik, Bd 1, sowie in Chmiel, Hammes and Bailey; Biochemical Engineering, beschrieben. Es kann sich um Batchverfahren, Fed-Batch, wiederholtes Fed-Batch oder kontinuierliche Fermentationsverfahren mit oder ohne Rückführung der Biomasse handeln. Hierbei wird zur Erhöhung der Ausbeute oft mit Luft, Sauerstoff, Kohlenmonoxid, Kohlendioxid, Ammoniak, Methan, Wasserstoff, Stickstoff oder geeigneten Gasmischungen durchströmt.

Die Fermentationsbrühe kann auch vorbehandelt werden, beispielsweise kann die Biomasse aus der Fermentationsbrühe entfernt werden. Hierzu können beispielsweise Methoden wie Filtration, Sedimentation und Flotation angewendet werden. Die Biomasse kann durch Zentrifugieren, Separatoren, Dekanter, Filter oder Entflotationsvorrichtungen entfernt werden. Zudem kann die Biomasse gewaschen werden, beispielsweise in Form einer Diafiltration, um die Produktausbeute zu maximieren. Die Fermentationsbrühe kann zudem konzentriert werden, beispielsweise durch Eindampfen bei geeigneten Bedingungen. Geeignete Verdampfer sind bekannt.

Die Fermentation kann erfindungsgemäß insbesondere zur Herstellung von Bernsteinsäure oder Salzen oder Derivaten davon eingesetzt werden. Geeignete Methoden sind beispielsweise in WO 2010/092155 auf Seiten 17 bis 19 und in den Beispielen beschrieben.

Nach der Fermentation, z. B. gemäß WO 2010/092155 A1, erfolgt im Allgemeinen die Abtrennung der Biomasse vom Produkt, beispielsweise durch Filtration. Dabei werden Feststoffe, insbesondere Zellen, abgetrennt. Produkt der Fermentation kann die Bernsteinsäure direkt sein, jedoch werden derzeit die höchsten Ausbeuten erzielt, wenn die Bernsteinsäure als Salz gewonnen wird, damit die Fermentation in einem pH-Bereich zwischen 6 und 8 ablaufen kann. Diese Salze sind beispielsweise Mono- und Disalze der Bernsteinsäure mit Ammoniak oder Aminen, sowie der Alkali- oder Erdalkalimetalle. Es sind auch Mischungen möglich. Aus diesen Salzen ist die Bernsteinsäure durch Ansäuern gewinnbar. Dies kann beispielsweise mithilfe von sauren lonentauschern geschehen, die anschließend im allgemeinen mit Mineralsäuren wie Salz- oder Schwefelsäure wieder regeneiert werden, oder durch Ansäuern mit Säuren wie Ameisensäure, Salzsäure, Schwefelsäure, Kohlensäure oder Phosphorsäure. Ebenfalls möglich ist auch die sog. Elektrodialyse, bei der mittels Strom und Membranen, wässrige Bernsteinsäurelösung und, je nach Gegenion der Bernsteinsäure z.B. Alkali- oder Erdalkalihydroxide entstehen, die in die Fermentation rückgeführt werden können.

In Abhängigkeit von den im Herstellungsverfahren anfallenden Verunreinigungen und der Aufarbeitung zur aufgereinigten Bernsteinsäure kann diese Verbindungen, die P, S, As, Sb, Bi, Sn oder Halogene wie Cl, Br und I enthalten, aufweisen.

Da die Fermentation in Wasser durchgeführt wird, fällt Bernsteinsäure bzw. deren Salze zumeist als wässrige Lösungen an. Das Ansäuern der Salze zur Gewinnung freier Bernsteinsäure ist zumeist ebenfalls ein wässriger Prozess unter Gewinnung von wässrigen Bernsteinsäurelösungen mit einem Gehalt von im Allgemeinen 1 bis 15 Gew.-% Bernsteinsäure, die je nach Konzentration ggf. temperiert werden muss, damit Bernsteinsäure nicht ausfällt, sofern man dieses nicht wünscht.

Es ist auch möglich, vor dem Ansäuern der Salze die Lösungen durch Entfernung von Wasser, beispielsweise durch Abdestillieren oder durch Pervaporation, aufzukonzentrieren. Anschließend kann die zumeist warme Salzlösung mit einem Gehalt von beispielsweise 10 bis 60 Gew.-% Bernsteinsäure-Salz bei Temperaturen von 20 - 100°C angesäuert werden. Anschließend kann abgekühlt werden, damit die Bernsteinsäure ausfällt. Die auskristallisierte Bernsteinsäure wird anschließend von der wässrigen Salzlösung, beispielsweise Na- oder Mg-Salze von Salz- oder Schwefelsäure, abfiltriert.

Ein erstes Kristallisat der Bernsteinsäure kann zur weiteren Aufreinigung, allerdings unter Verlusten an Produkt durch Ausschleusungen, ein weiteres Mal in Wasser aufgelöst und wieder kristallisiert werden. Dies kann so oft geschehen, bis man die Bernsteinsäure bis zur gewünschten Spezifikation aufgereinigt hat, allerdings nimmt die Ausbeute mit jedem Kristallisationsschritt ab. Im erfindungsgemäßen Verfahren wird bevorzugt maximal zwei Mal kristallisiert, besonders bevorzugt maximal ein Mal. Dies auch deshalb, weil Kristallisationen nicht nur die Ausbeute vermindern, sondern auch die Investitionen hierfür beträchtlich sind.

Die auf diese Weise hergestellt Bernsteinsäure enthält im Allgemeinen folgende Verunreinigungen, wobei die Menge an Verunreinigungen mit der Anzahl an Reinigungsschritten abnimmt, wobei nachstehend mit HCl angesäuert wurde und sich die %-Angaben auf Gew.-% bzw. Gew.-ppm berechnet auf das jeweilige Element beziehen.

### Wässrige Bernsteinsäurelösung nach Ansäuern mit HCl

Kristalle nach 1. Kristallisation:
Summe aus Halogen berechnet als Chlor 0,01 bis 2 %
Schwefel 0,001 bis 0,1 %
Stickstoff 0,001 bis 0,1 %
Phosphor 0,01 bis 100 ppm
Arsen, Antimon, Wismuth, Zinn in Summe 0,01 bis 20 ppm
Magnesium 0,1 bis 1000 ppm
Eisen, Mangan, Chrom, Molybdän in Summe 0,1 bis 100 ppm
Calcium 0,1 bis 100 ppm
Natrium, Kalium in Summe 0,1 bis 100 ppm

Kristalle nach 2. Kristallisation:
Summe aus Halogen berechnet als Chlor 0,01 bis 20 ppm
Schwefel 0,01 bis 10 ppm
Stickstoff 0,01 bis 10 ppm
Phosphor 0,01 bis 3 ppm
Arsen, Antimon, Wismut, Zinn in Summe 0,01 bis 3 ppm
Magnesium 0,01 bis 3 ppm
Eisen, Mangan, Chrom, Molybdän in Summe 0,01 bis 3 ppm
Calcium 0,01 bis 3 ppm
Natrium, Kalium in Summe 0,01 bis 3 ppm

Erfindungsgemäß liegt vorzugsweise der Schwefelgehalt, bezogen auf Bernsteinsäure, am Ende von Schritt a) im Bereich von 0,001 bis 0,1 Gew.-% oder 0,01 bis 10 ppm.

Vorzugsweise wird in Schritt a) Bernsteinsäure fermentativ aus mindestens einer Kohlenstoffquelle hergestellt und nach Abtrennen der Biomasse aus der Fermentationsbrühe durch Ansäuern in die Säureform überführt. Besonders bevorzugt wird die so hergestellte Bernsteinsäure ohne weitere/zusätzliche Reinigungsschritte in Schritt b) überführt.

In den nachfolgenden Schritten angegebene Temperaturangaben beziehen sich, sofern nicht anders angegeben, auf den Sumpf der jeweiligen Verdampfung oder Destillation. Angegebene Drücke beziehen sich, sofern nicht anders angegeben, auf den Kopf der Destillation (Kopfdruck). Bei einfachen Verdampfungen handelt es sich um den Druck in der Verdampfungsstufe.

### Schritt b)

Im erfindungsgemäßen Verfahren wird nun Bernsteinsäure als wässrige Lösung oder als Schmelze, die noch Wasser enthalten kann, in den erfindungsgemäßen Schritt b) eingebracht. Je nach Wassergehalt kann in einem ersten Schritt Lösungswasser entfernt werden, wobei dabei bereits die Umwandlung von Bernsteinsäure ins Anhydrid ablaufen kann, wobei dabei wiederum Wasser freigesetzt wird, das in diesem Schritt dann mit abgetrennt wird. Bevorzugt wird eine Bernsteinsäurelösung mit einer Konzentration von 5 - 50 Gew.-% in eine oder mehrere hintereinander oder parallel geschaltete Verdampfungsvorrichtungen eingebracht, in der vorzugsweise bei 100 bis 250°C (Temperatur gemessen im Sumpf) und vorzugsweise bei Drücken von 50 bis 1000 mbar absolut Wasser abdestilliert wird. Bevorzugt sind 150 bis 220°C, besonders bevorzugt 150 bis 200°C bei bevorzugten Drücken von 0,1 bis 0,5 bar absolut, besonders bevorzugt bei 0,15 bis 0,3 bar absolut. Zur optimalen Energieausnutzung können die Verdampfungseinrichtungen, ggf. mit anderen Anlageneinheiten, in einem Wärmeverbund gekoppelt sein. Auf der Verdampfungsvorrichtung kann eine Kolonne aufgesetzt sein, die durch Rücklauf den Verlust an Bernsteinsäure bzw. Anhydrid vermeidet. Die Verdampfervorrichtung kann beispielsweise ein einfacher Kessel sein, der gerührt und/oder umgepumpt werden kann. Möglich ist ebenfalls ein Fallfilm-, Dünnschicht-, Naturumlauf-, Zwangsumlauf- oder Wendelrohrverdampfer. Das Sumpfprodukt, in dem sich bereits gebildetes Bernsteinsäureanhydrid befindet und das einen Wassergehalt von vorzugsweise 0,01 bis 30 Gew.-%, bevorzugt 0,05 bis 15 Gew.% besonders bevorzugt 0,1 bis 10 Gew.-% aufweist, kann nun als solches in den Schritt c) überführt werden, oder es kann in einer weiteren Destillationseinheit weiter umgesetzt und aufkonzentriert werden.

### Schritt c)

In Schritt c) wird das Bernsteinsäureanhydrid aus Schritt b) in die Gasphase überführt.

Vorzugsweise kann Schritt c) in mindestens einer Destillationsvorrichtung bei einem Kopfdruck im Bereich von 0,02 bis 2 bar und einer Sumpftemperatur im Bereich von 100 bis 300°C unter Abtrennung von Hochsiedern über den Sumpf durchgeführt werden. Bevorzugt wird in dieser Ausführungsform Schritt c) bei niedrigerem Druck als Schritt b) durchgeführt, und in Schritt c) werden Wasser und gegebenenfalls Leichtsieder über Kopf abgetrennt, und gasförmiges Bernsteinsäureanhydrid wird über einen Seitenabzug gewonnen.

Alternativ können Schritt b) und Schritt c) zusammengefasst und in mindestens einer Destillationsvorrichtung bei einem Kopfdruck im Bereich von 0,02 bis 2 bar und einer Sumpftemperatur im Bereich von 100 bis 300°C, bevorzugt siehe unten unter Abtrennung von Hochsiedern über den Sumpf, Abtrennung von Wasser über Kopf und Gewinnung von gasförmigem Bernsteinsäureanhydrid über einen Seitenabzug durchgeführt werden. Nachstehend sind die einzelnen Verfahrensalternativen näher erläutert.

Zusammen mit dem Wasser werden in diesem Schritt bevorzugt schädliche Carbonsäuren wie v.a. Ameisensäure und Essigsäure entfernt, damit diese später nicht durch Korrosion am Katalysator diesen schädigen können.

In dieser weiteren (oder mehreren hintereinander oder parallel geschalteten) Destillationseinheit wird Bernsteinsäureanhydrid, das noch Bernsteinsäure enthalten kann, weiter aufgereinigt bzw. hergestellt. Entscheidend dabei ist, dass Bernsteinsäureanhydrid in die Gasphase gebracht wird, damit es bevorzugt von hochsiedenden Verunreinigungen abgetrennt werden kann. Damit der Reinigungseffekt möglichst hoch ist, wird die Destillation vorzugsweise mit Rücklauf betrieben. Bevorzugt beträgt der Rücklauf bezogen auf die hinzugeführte Menge an Bernsteinsäure/Bernsteinsäureanhydrid zwischen 0,1 und 10 Mengeneinheiten, besonders bevorzugt 0,2 bis 5 Mengeneinheiten. Die Hochsieder werden über Sumpf ausgeschleust.

Bei dieser Stufe gibt es zwei bevorzugte Verfahrensvarianten: Gemäß einer Variante erfolgt die Trennung der Hochsieder von gasförmigem Bernsteinsäureanhydrid bei 100 bis 300°C, bevorzugt 150 bis 270°C, besonders bevorzugt 170 bis 250°C (Sumpftemperaturen) und (Kopf)Drücken von 0,02 bis 2 bar absolut, vorzugsweise von 0,03 bis 1 bar und besonders bevorzugt von 0,04 bis 0,5 bar. Dabei wird das gasförmig erzeugte Bernsteinsäureanhydrid für die Herstellung von THF vorzugsweise nicht kondensiert und aus der Destillationsvorrichtung ausgeschleust. Anschließend wird es in die erfindungsgemäße Stufe c) überführt.

Gemäß der anderen Variante, die die Zusammenschaltung/Kombination der erfindungsgemäßen Schritte c) bis e) umfasst, erfolgt das Verdampfen von Bernsteinsäureanhydrid in Gegenwart von Wasserstoff, wobei ein Teil des gasförmigen Bernsteinsäureanhydrids zur Erzeugung des Rücklaufes kondensiert. Der Rest gelangt zusammen mit dem Wasserstoff über das Schutzbett in die Hydrierung. Dies wird im allgemeinen bei Sumpftemperaturen von 150 bis 300°C, bevorzugt 160 bis 270°C, besonders bevorzugt 180 bis 250°C bei Drücken (absolut) von 1 bis 65 bar, bevorzugt 2 bis 30 bar, besonders bevorzugt bei 5 bis 20 bar durchgeführt. In einer bevorzugten Variante wird das Bernsteinsäureanhydrid zusammen mit einem höher als Bernsteinsäureanhydrid siedenden Lösemittel dem Wasserstoffstrom zur Verdampfung des Bernsteinsäureanhydrids ausgesetzt. Dies hat den Vorteil, dass beispielsweise eine Kolonne zum Austreiben oder Strippen des Anhydrids effizienter betrieben werden kann, da auch bei abnehmender Konzentration an Anhydrid die Kolonnenböden durch das hochsiedende Lösemittel benetzt werden. Dabei wird das Lösemittel bevorzugt über die Stripp-Kolonne im Kreis gefahren. Beispiele für dieses Lösungsmittel, das bevorzugt gegenüber Beinsteinsäureanhydrid und Wasserstoff inert sein sollte, sind Phthalate oder Terephthalate auf Basis C₄ bis C₁₅-Alkoholen, beispielsweise Dibutylphthalat, entsprechend kernhydrierte Phthalate oder Terephthalate, Kohlenwasserstoffe, Ether auf Basis Ethylenoxid und/oder Propylenoxid und dergleichen.

Zur Steigerung der Ausbeute kann der Bernsteinsäureanhydrid enthaltende Sumpfstrom ganz oder zumindest teilweise in eine oder mehrere vorhergehende Stufen rückgeführt werden. Jedoch wird vor allem bei länger laufenden Prozessen zur Herstellung technischer Produktmengen an THF, eine Ausschleusung zur Vermeidung von Aufpegelungen vorteilhaft sein. Deshalb ist es bevorzugt, diesen Ausschleusestrom in einer weiteren Destillationseinrichtung weiter aufzuarbeiten. Bevorzugt wird hierbei ein Dünnschichtverdampfer eingesetzt, in dem Bernsteinsäureanhydrid über Kopf abdestilliert, das dann in eine der vorhergehenden Stufen rückgeführt wird. Der Hochsiederstrom wird ausgeschleust. Die Verdampfung wird bei vorzugsweise 100 bis 300°C, bevorzugt 150 bis 270°C, besonders bevorzugt bei 180 - 250°C und Drücken von vorzugsweise 1 bis 200 mbar absolut, bevorzugt zwischen 3 und 100 mbar, besonders bevorzugt bei 5 bis 50 mbar durchgeführt.

Um im erfindungsgemäßen Schritt c) schädliche, basische N-haltige Verbindungen zu entfernen, ist es bevorzugt, die basischen Verbindungen in hochsiedende Stoffe zu überführen. Diese basischen N-haltigen Verbindungen können beispielsweise Ammoniak, aliphatische Amine, Aminosäuren usw. sein. Um zu verhindern, dass diese zusammen mit Bernsteinsäureanhydrid in die Gasphase übergehen, ist es vorteilhaft, diese in hochsiedende Verbindungen zu überführen. Dies kann beispielsweise dadurch geschehen, indem man in Gegenwart einer Säure arbeitet, die zusammen mit den basischen Verbindungen hochsiedende Salze bildet. Dies sind beispielsweise Salze von hochsiedenden Carbonsäuren wie Adipinsäure, saure Aminosäuren, Sulfonsäuren wie Dodecylbenzolsulfonsäure, Methansulfonsäure, Mineralsäuren wie Phosphorsäure, Schwefelsäure oder Heteropolysäuren wie Wolframatophosphorsäure. Bevorzugt wird pro Äquivalent basischer Verbindung 1 bis 1,5 Mol-Äquivalent Säure zugegeben. Eine weitere bevorzugte, ggf. zur Salzbildung ergänzende Möglichkeit, ist die chemische Umsetzung von basischen N-haltigen Verbindungen, in hochsiedende Verbindungen, z.B. die Bildung von Amiden. Dazu werden sie z.B. zu Sulfonsäure- oder Carbonsäureammoniumsalzen bei Verweilzeiten von 0,1 bis 2 h und Temperaturen von 150 - 300°C umgesetzt. Dies kann beispielsweise in den Sümpfen der Verdampfungseinrichtungen ablaufen. Geeignete Reaktionspartner sind z.B. Sulfonsäuren oder Carbonsäuren wie oben beschrieben. Werden basische N-haltige Komponenten nicht weitestgehend vor der Hydrierung entfernt, so muss gegebenenfalls damit gerechnet werden, dass die für die Herstellung von THF notwendigen sauren Katalysatorzentren nach und nach neutralisiert werden und dadurch die Ausbeute an THF reduziert wird.

### Schritt e)

Im erfindungsgemäßen Schritt e) wird gasförmiges Bernsteinsäureanhydrid zusammen mit Wasserstoff einer Gasphasen-Hydrierung zugeführt. Dabei beträgt das molare Verhältnis von Wasserstoff zu Bernsteinsäureanhydrid vorzugsweise von 20 - 300 zu 1, bevorzugt 30 - 250 zu 1, besonders bevorzugt 50 - 200 zu 1. Die Drücke (absolut) liegen vorzugsweise bei 1 bis 65 bar, bevorzugt bei 2 bis 30 bar, besonders bevorzugt bei 5 bis 20 bar. Die Temperaturen betragen vorzugsweise 150 bis 350°C, bevorzugt 170 bis 320°C, besonders bevorzugt 200 bis 300°C.

Die zur Hydrierung genutzten Heterogen-Katalysatoren haben als Hydriermetall vorzugsweise zumindest eines der Elemente ausgewählt aus Ru, Re, Co und Cu. Bevorzugte Katalysatoren enthalten zumindest Cu. Der Gew.-% Anteil des Hydriermetalls am Gesamtgewicht des Katalysators (berechnet als Element) liegt vorzugsweise zwischen 0,5 und 80 %. Im Falle von Cu liegt der bevorzugte Anteil zwischen 10 und 80 %, besonders bevorzugt zwischen 25 und 65 %.

Die Hydriermetalle sind bevorzugt auf einem Trägersystem aufgebracht. Geeignete Träger weisen vorzugsweise saure Zentren auf und enthalten vorzugsweise Oxide basierend auf B, Al, Si, Ti, Zr, La, Ce, Cr, oder Kohlenstoff, beispielsweise in Form von Aktivkohle. Ein weiterer Träger, der nicht oxidisch vorliegt, ist beispielsweise SiC.

Die Herstellung der Katalysatoren wird beispielsweise durch Tränken von Aktivmetallvorläufern, z.B. Cu-Salzlösungen, auf den Trägern erreicht. Geeignet sind auch Fällkatalysatoren, bei denen die Aktivkomponenten auf einen Träger aufgefällt werden oder zusammen mit dem Trägermaterial aus gelösten Vorstufen desselben gefällt werden. Nach Trocknung und ggf. Kalzination des Katalysatormaterials wird bevorzugt vor Beginn der Hydrierung der Katalysator mit Wasserstoff aktiviert.

Besonders bevorzugte Katalysatoren enthalten neben Cu auch Aluminiumoxid.

Die Heterogen-Katalysatoren sind im Allgemeinen Formkörper mit einer durchschnittlichen Teilchengröße, die über einem Millimeter liegt. Bevorzugt werden Stränge, Tabletten, Sternstränge, Triloben, Hohlkörper usw. verwendet.

Als Reaktoren für die Hydrierung kommen dem Fachmann bekannte Typen zum Einsatz. Beispiele hierfür sind Schachtreaktoren, Rohrbündelreaktoren, Wirbelschichtreaktoren usw. Es kann in einem Reaktor hydriert werden oder in mehreren Reaktoren parallel oder hintereinander angeordnet, auch mehrere Typen miteinander kombiniert. Der Umsatz an Bernsteinsäureanhydrid zum Ende der Reaktoren beträgt vorzugsweise > 95 %, bevorzugt sind > 99%, besonders bevorzugt > 99,9 %. Nach dem Reaktor wird der produktführende Gasstrom vorzugsweise auf unter 60°C zum Auskondensieren von THF abgekühlt. Bevorzugt sind unter 40 °C, besonders bevorzugt unter 20°C. Dies kann auch mehrstufig ablaufen, wobei die Temperatur durch den Einsatz mehrerer Kühler entlang des Gasstroms abnimmt. Bevorzugt wird die Hydrierung mit Kreisgas betrieben. Hierfür wird der vom Produkt möglichst weitgehend befreite Gasstrom über einen Kreisgaskompressor in die Reaktion rückgeführt, wobei er bevorzugt dazu verwendet wird, Bernsteinsäureanhydrid zu verdampfen. Der durch die Hydrierung und ggf. Verluste über Abgas bzw. im flüssigen Austrag gelöstes Gas verbrauchte Wasserstoff wird entsprechend ergänzt. Wenn mit Abgas zur Ausschleusung eventueller Inerte wie z.B. Stickstoff und Argon, die zusammen mit dem Wasserstoff eingebracht werden, oder entstehender Verbindungen wie z.B. Methan oder Kohlendioxid gearbeitet wird, dann wird vorzugsweise weniger als 10 % bevorzugt weniger als 5 % besonders bevorzugt weniger als 3 % der Menge, bezogen auf zugefahrenen Frischwasserstoff, ausgeschleust.

### Schritt d)

Der vorgelagerte erfindungsgemäße Schritt d) ist maßgeblich dafür verantwortlich, dass der Hydrierprozess möglichst lange bei hoher Selektivität und hohem Umsatz betrieben werden kann. Dies wird ermöglicht, indem Katalysatorgifte, die flüchtige Verbindungen bilden und zusammen mit Bernsteinsäureanhydrid in die Hydrierung gelangen könnten und z. B. die Elemente P, S, As, Sb, Bi, Sn oder Halogene wie Cl, Br und I, allen voran Schwefel, enthalten, auf einem Katalysator oder Absorber, hier auch als Schutzbett (guard bed) bezeichnet, aufgenommen werden.

Der Katalysator, der diese Katalysatorgifte aufnehmen soll, ist nach Möglichkeit ein Katalysator, der Bernsteinsäureanhydrid nicht schädigt, sondern im Gegenteil, bevorzugt bereits hydrierend in Richtung gewünschtes Produkt wirkt und, nach Aufnahme der Katalysatorgifte, zwar in seiner Hydrieraktivität nachlassen darf, jedoch nicht zersetzend auf das Anhydrid oder über etwaiges im Kreisgas mitgeführtes Produkt, z.B. THF einwirkt. Bevorzugt weist der Katalysator ein möglichst scharfes Profil bei hohem Aufnahmevermögen bzgl. der Katalysatorgifte auf. Dabei bedeutet scharfes Profil, dass die genannten Katalysatorgifte im Schutzbett in einem räumlich sehr engen Bereich absorbiert werden und keine breite Verteilung über die Länge des Schutzbetts aufweisen. Hierdurch ist es möglich, verbrauchtes Schutzbett gezielt auszutauschen, wobei nur geringe Mengen an Schutzbett ausgetauscht werden müssen. Bevorzugt ist, dass, gemessen am Verhalten des Schwefels, bei Erreichen von mindestens 90 % der Aufnahmekapazität unter Reaktionsbedingungen, nach weiteren 50 cm, bevorzugt 40 cm, besonders bevorzugt 30 cm entlang des Hydrierweges bzw. Schutzbettes erst 10 % Schwefel aufgenommen worden ist. Dabei ist zu beachten, dass Katalysatoren, die bedingt durch ihren Herstellprozess beispielsweise Sulfat enthalten, die Messungen verfälschen können. In diesem Fall ist es notwendig, den "Nullwert" an Schwefel entsprechend von dem Wert, der durch den Schwefel im Hydrierfeed verursacht wird, abzuziehen.

Geeignete Katalysatoren zur Entfernung von P, S, As, Sb, Bi, Sn und Halogenen wie Cl, Br und I, insbesondere Schwefel, enthalten z. B. Mo, Co, Ru, Re und Cu, sofern sie nicht auch schon hydrierend wirken sollten, beispielsweise ZnO. Bevorzugt sind Ru und Cu, besonders bevorzugt ist Cu. Dabei ist es vorteilhaft, wenn der Gehalt an Metall, das die Katalysatorgifte aufnehmen kann, möglichst hoch ist. So ist der Gehalt der zur Aufnahme der giftigen Bestandteil, gemessen als Element vorzugsweise größer als 10 % bzgl. des Gesamtgewichtes des Katalysators, bevorzugt > 25 %, besonders bevorzugt > als 40 %, jedoch vorzugsweise nicht über 90 %, da sonst die zur Aufnahme befähigte Oberfläche zu klein wird. Die Aufnahmekapazität an Schwefel liegt normiert auf Metallgehalt vorzugsweise zwischen 0,5 und 10 Gew.-% bevorzugt zwischen 1 und 10 Gew.-%.

In einer besonders bevorzugten Ausführungsform enthält der Katalysator zur Entfernung von Katalysatorgiften die gleichen Bestandteile wie der eigentliche Hydrierkatalysator, wobei im Idealfall, zur Vermeidung von Verwechslungen bei der Beschickung der Apparate, oder zusätzlichen Katalysatorproduktionsaufwand, der gleiche Katalysator für Hydrierung und Entgiftung eingesetzt wird.

Der Katalysator zum Entfernen von Katalysatorgiften wird vorzugsweise als fest angeordnete Schicht z.B. in einem Schacht- oder Rohrbündelreaktor eingesetzt. Dies kann räumlich vom eigentlichen Hydrierkatalysator getrennt, d.h. in zwei Apparaten, zur Vermeidung von zu vielen Apparaten aber bevorzugt in einem Reaktor, zusammen mit dem Hydrierkatalysator erfolgen. Unabhängig davon, ob nur ein oder mehrere Apparate verwendet werden, besteht die Möglichkeit, mit möglichst wenig Katalysator zur Entfernung von Katalysatorgiften zu arbeiten, wenn dieser, möglichst bevor er vollständig beladen ist, von Zeit zu Zeit entfernt und wieder ergänzt wird. Dies ist dann sinnvoll, wenn der erfindungsgemäße Prozess beispielsweise wegen Wartungsarbeiten ohnehin abgestellt werden muss.

Das Volumen-Verhältnis von eigentlichem Hydrierkatalysator zu Katalysator, der die Gifte entfernt, ist vorzugsweise 3 - 200 zu 1, bevorzugt 5 - 100 zu eins, besonders bevorzugt 10 - 50 zu 1.

Der Katalysator zur Entfernung von Giften, wird vor seiner Verwendung bevorzugt mit Wasserstoff bei Temperaturen und Bedingungen analog dem eigentlichen Hydrierkatalysator aktiviert. Auf diese Weise kann eine Lebensdauer des Hydrierkatalysators von mehr als 6 Monaten, bevorzugt mehr als 1 Jahr, erreicht werden.

Die Entfernung von Schwefel-Verbindungen und anderen Katalysatorgiften kann auch in der Flüssigphase erfolgen. Dies ist dann vorteilhaft, wenn weitere Prozessschritte, die zu den gewünschten Produkten führen, in der Flüssigphase erfolgen, beispielsweise die Hydrierung oder eine Veresterung von Bernsteinsäureanhydrid bzw. Bernsteinsäure zu Bernsteinsäurediester wie z.B. Bernsteinsäuredimethylester, der dann in der Gasphase hydriert wird.

Die Katalysatoren sind die gleichen, wie sie auch in der Gasphase angewandt werden können und sind, sofern sie metallischer Natur sind, zuvor beispielsweise mit Wasserstoff aktiviert worden. Die Temperaturen zur Entfernung unerwünschter Nebenkomponenten in flüssiger Phase liegen im Bereich von 50 - 250°C, bevorzugt 70 bis 230°C, besonders bevorzugt 90 bis 210°C. Die Drücke sind im Prinzip unkritsch, solange kein Sieden erfolgt. Sie liegen zwischen 0,5 und 300 bar absolut. Sofern metallische Materialien verwendet werden, erfolgt die Entfernung der unerwünschten Komponenten bevorzugt in Gegenwart von Wasserstoff.

Es ist auch möglich, mit reines Anhydrid zu behandeln, sondern es in inerten Lösemitteln zu lösen oder in Reaktionsprodukten wie THF, gamma-Butyrolacton oder Butandiol oder in Methanol oder in Wasser.

### Schritt f)

Vorzugsweise umfasst das Verfahren auch den nachgelagerten Schritt f), umfassend die destillative Abtrennung von Wasser und Hochsiedern vom Tetrahydrofuran, 1,4-Butandiol und/oder gamma-Butyrolacton. Bevorzugt wird Schritt f) bei der Herstellung von Tetrahydrofuran in mindestens drei Destillationskolonnen durchgeführt, wobei
f1) in einer ersten Destillationskolonne im Sumpf Hochsieder abgetrennt werden und über Kopf ein Tetrahydrofuran/Wasser-Azeotrop gewonnen wird,
f2) das Tetrahydrofuran/Wasser-Azeotrop aus Schritt f1) in einer zweiten Kolonne, die bei höherem Druck als die erste Kolonne in Schritt f1) betrieben wird, aufgetrennt wird in ein Tetrahydrofuran/Wasser-Azeotrop, das über Kopf abgetrennt und vorzugsweise in Schritt f1) zurückgeführt wird, und Tetrahydrofuran, das über den Sumpf gewonnen wird und
f3) das in Schritt f2) über den Sumpf gewonnene Tetrahydrofuran in einer dritten Kolonne von Hochsiedern, die über den Sumpf ausgetragen werden, befreit wird.

Bevorzugte Ausführungsformen des Schritts f) sind nachstehend näher erläutert.

Der erfindungsgemäße Schritt f) beinhaltet die Aufreinigung des Hydrieraustrages. Dieser enthält überwiegend die beiden Hydrierprodukte THF und Wasser, daneben in kleinen Mengen, bezogen auf das Produkt THF, vorzugsweise molar weniger als 7 % n-Butanol, bevorzugt weniger als 5 %, vorzugsweise weniger als 2 %, bevorzugt weniger als 1 % gamma-Butyrolacton, sowie weitere Produkte, zumeist aber unter 1 % molar bezogen auf THF, bevorzugt unter 0,5 % wie n-Propanol, Methanol, Buttersäure, n-Butyraldehyd, n-Butylmethylether und andere, mengenmäßig unbedeutende Verbindungen. Die Ausbeute an THF bezogen auf eingesetztes Bernsteinsäureanhydrid beträgt über die gesamte Laufzeit des Hydrierkatalysators vorzugsweise über 90 %, bevorzugt über 95 %, besonders bevorzugt über 96 %.

Der Hydrieraustrag wird in einer ersten Kolonne von Hochsiedern befreit, wobei über Kopf ein THF/Wasser-Azeotrop, das ggf. noch n-Butyraldehyd und andere Leichtsieder enthalten kann und über Sumpf Wasser, n-Butanol und, wenn entstanden, gamma-Butyrolacton entfernt wird. Dieses Sumpfprodukt kann separat aufgetrennt werden, um Butanol und gamma-Butyrolacton zu gewinnen, wobei Letzteres in die Hydrierung zurückgeführt werden kann. Das Kopfprodukt wird in einer weiteren Kolonne, die bevorzugt bei höherem Druck betrieben wird als die erste Kolonne, eingebracht. Hier wird wiederum ein THF/Wasser-Azeotrop über Kopf abgetrennt, diesmal aber, aufgrund des höheren Druckes, mit einem höheren Wasseranteil. Dieses Azeotrop wird bevorzugt in die erste Kolonne rückgeführt. Sofern Methanol in der Hydrierung entstanden sein sollte, kann dieses in dieser Kolonne zusammen mit etwas THF über Kopf ausgeschleust werden, wobei dann das THF/Wasser-Azeotrop bevorzugt über einen Seitenabzug im Verstärkungsteil gewonnen wird. Das Sumpfprodukt der zweiten Kolonne, nahezu wasserfreies THF (< 1000 ppm Wasser), wird als solches bereits weiter verwendet, oder aber in einer dritten Kolonne nochmals "feindestilliert", beispielsweise zur Ausschleusung von eventuellen Hochsiedern wie n-Butyraldehyd. Die erste Kolonne mit vorzugsweise 10 bis 80, bevorzugt 40 bis 60 theoretischen Stufen wird bei einem Absolutdruck von vorzugsweise 0,5 bar bis 4 bar, bevorzugt 1 bar bis 3 bar, die zweite Kolonne mit vorzugsweise 10 bis 70, bevorzugt 40 bis 60 theoretischen Stufen bei einem Absolutdruck von vorzugsweise 5 bar bis 20 bar, bevorzugt 6 bar bis 12 bar betrieben. Die Kolonnen können unterschiedliche Einbauten wie z. B. Füllkörper, Blechpackungen, Gewebepackungen oder Böden aufweisen.

Die Aufarbeitung des Hydrieraustrags kann auch wie in DE-A-3726805 oder in WO03/074507 offenbart erfolgen. Alternative Aufreinigungskonzepte beinhalten beispielsweise die Abreicherung von Wasser mittels Membranfiltration. Ebenfalls möglich ist die Wasserentfernung mittels konzentrierter Natron- oder Kalilauge. Nach diesen Wasserabtrennmethoden wird das THF vorzugsweise in zumindest einer Kolonne weiter aufgereinigt.

Vorstehend sind bevorzugte Verfahrensvarianten in Bezug auf die Herstellung von Tetrahydrofuran (THF) beschrieben.

Sofern 1,4-Butandiol das Produkt der Wahl ist, so wird in einem ersten Destillationsschritt Wasser und Nebenprodukte wie Alkohole, z.B. n-Butanol, n-Propanol sowie THF vom Butandiol abdestilliert. In einem zweiten Schritt wird das Butandiol weiter gereinigt, indem es beispielsweise in eine Kolonne mit Seitenabzug eingebracht wird und gegenüber Butandiol leichtersiedende Komponenten wie gamma Butrolacton über Kopf abgetrennt werden, über Seitenstrom Butandiol gewonnen wird und über Sumpf Hochsieder ausgeschleust werden. Hochsieder und gamma-Butyrolacton können, zumindest zum Teil, beispielsweise zu über 50 %, wieder in die Hydrierung eingeschleust werden. Anstatt einer Kolonne mit Seitenabzu können auch zwei voneinander getrennte Kolonnen verwendet werden, wobei Butandiol als Reinprodukt über Kopf der zweiten Kolonne gewonnen wird.

Ist gamma-Butyrolacton das Produkt, so orientiert sich die destillative Reinigung am selben Schema wie bei Butandiol.

Die Erfindung wird durch die nachstehenden Beispiele näher erläutert.

### Beispiele

### Beispiel 1: Gewinnung von Bernsteinsäureanhydrid (BSA)

Ein nach WO 2010/092155 A1, Beispiel 6, erhaltener Roh-Fermentationsaustrag wurde nach Abtrennung der Bio-Masse durch Filtration mit HCl bis zu einem pH von 3 angesäuert. Dieses Gemisch wurde kontinuierlich in einen umgepumpten Verweilzeitbehälter mit aufgesetzter Kolonne gepumpt. Bei ca. 4 Stunden mittlerer Verweilzeit wurde dann Wasser bei 200 mbar und 180°C Sumpftemperatur abdestilliert. Der flüssige, ca. 180°C heiße Sumpf, der Hochsieder, BSA und unter 5 % freie Bernsteinsäure enthielt, wurde kontinuierlich in eine Kolonne mit Seitenabzug im Verstärkungsteil eingeführt und bei einem Kopfdruck von 50 mbar und Sumpftemperaturen von ca. 180 °C aufdestilliert. Über Kopf wurde im Wesentlichen Wasser entfernt, welches einen Gehalt an 2 Gew.-ppm N enthielt, und über den Seitenabzug wurde BSA und über den Sumpf wurden die Hochsieder ausgeschleust. In den Hochsiedern fanden sich 0.03 Gew.-% N, 0.02 Gew.-% S. Auf diese Weise ließ sich BSA bezogen auf Bernsteinsäure im Fermentationsaustrag mit ca. 97 % Ausbeute erhalten. Das BSA wies einen Gehalt an Schwefel von 5 Gew.-ppm auf.

### Beispiel 2a: Hydrierungen zu THF

Die in den Beispielen verwendete Apparatur bestand aus einem begleitbeheizten Zulaufteil mit Vorratsgefäß und Pumpe, einem Verdampfer gefüllt mit Glasringen, einem 3 m langen Rohrreaktor mit 2,7 cm Innendurchmesser und außenliegender Doppelmantelölbeheizung bzw. Kühlung sowie innenliegendem Thermoelementrohr, einem wassergekühltem ersten Abscheider, einem zweiten auf 6°C gekühlten Abscheider sowie einem Kreisgasgebläse sowie Frischgas- und Abgas-Einrichtungen. Zur Verdampfung von Bernsteinsäureanhydrid wurden das Bernsteinsäureanhydrid (BSA), das Kreisgas und der Frischwasserstoff in den Verdampfer geleitet. Das Mol-Verhältnis von Frischwasserstoff zu BSA betrug 2,1 zu 1, wobei das überschüssige Gas als Abgas ausgeschleust wurde. Das Molverhältnis von Kreisgas zu BSA betrug ca. 100 zu 1.

### Vergleichsbeispiel 2b:

Der Reaktor wurde mit 1 Liter eines CuO (50 Gew.-%)/Al₂O₃-Katalysators (2,5 mm Stränge) gefüllt. Oberhalb des Katalysators wurden 100 ml Glaskugeln als Inertschüttung gefüllt. Nach Inertisierung mit Stickstoff wurde der Katalysator mit einer Stickstoff/Wasserstoff-Mischung bei Normaldruck aktiviert (Der Gasstrom wird auf 99,5 % Stickstoff und 0,5 % Wasserstoff eingestellt, dann der Reaktor aufgeheizt auf 130°C. Nach 2 Stunden wird der Reaktor in 5°C-Stufen weiter aufgeheizt, wobei jede Temperatureinstellung für 30 Minuten gehalten wird. Nach Erreichen von 180°C wird der Wasserstoffgehalt auf 1 % erhöht, nach einer Stunde auf 5 % wiederum für eine Stunde, danach wird der Wasserstoffgehalt auf 100% angehoben). Nach Aktivierung des Katalysators wird das Kreisgasgebläse in Betrieb genommen und der Druck im Reaktor auf 9 bar absolut sowie die Reaktortemperatur auf 230°C eingestellt.

Anschließend wurde der BSA-Zulauf oben in den Reaktor gestartet. Es wurden kontinuierlich 100 g BSA/h gefördert. Daraufhin erhöhte sich die Temperatur im ersten Drittel des Reaktors auf bis zu 245°C, sank dann auf nahezu Ölbeheizungs/Kühlungsniveau ab (ca. 230°C), um dann im letzten Drittel der Katalysatorschüttung wieder auf bis zu 235°C anzusteigen und kurz vor Ende der Katalysatorschüttung wieder auf nahezu 230°C abzufallen.

Die in den Abscheidern anfallenden flüssigen Reaktionsausträge wurden gesammelt und vereinigt und gaschromatographisch (GC-Flächenprozent) analysiert. Es fanden sich 98,3 % THF und 1,5 %n-Butanol. Der Rest bestand aus mehreren Verbindungen, als Einzelkomponente nicht über 0,05 %, wie n-Butyraldehyd, Dibutylether und gamma-Butyrolacton.

Nach einer Laufzeit von 1000 h hatte sich das Temperaturprofil im Reaktor dahingehend verändert, dass die Orte der höchsten Temperaturen sich etwas nach hinten verschoben hatten und die Reaktionstemperatur am Ende des Reaktors bei ca. 232°C lag, also nicht mehr das Ölbeheizungs/Kühlungsniveau erreichte. Im Hydrieraustrag fanden sich 95,1 % THF, 1,8 % n-Butanol, 2,8 % gamma-Butyrolacton, 0,1 % BSA, sowie jeweils unter 0,05 % z.B. n-Butyraldehyd, Buttersäure, Dibutylether und Methylbutylether.

Kurze Zeit darauf musste die Anlage abgestellt werden, da, vermutlich durch Belag von BSA, die Abscheider zu verstopfen drohten.

### Erfindungsgemäßes Beispiel 2b:

Das Vergleichsbeispiel 2 wurde wiederholt, mit dem Unterschied, dass oberhalb der ein Liter Katalysator statt 100 ml Glaskugeln in dieser Reihenfolge 10 ml Glaskugeln, 50 ml CuO (60 Gew.-%)/Al₂O₃ 3 mm Tabletten, und 40 ml Glaskugeln eingefüllt wurden. Die Glaskugeln zwischen den beiden Cu-Katalysatoren dienten dazu, diese, zwecks angestrebter Analyse, einfacher separat ausbauen zu können. Die Einfüllhöhe der 50 ml Katalysator betrug unter Berücksichtigung des Reaktorinnendurchmessers und des innenliegenden Rohres mit den Thermoelementen ca. 10 cm.

Nach 2000 h Versuchszeit wurde der Versuch abgebrochen, ohne dass sich an den Temperaturprofilen bzw. der Austragszusammensetzung Wesentliches geändert hätte. So fanden sich im Austrag 98,2 % THF, 1,6 %n-Butanol. Der Rest bestand aus mehreren Verbindungen, als Einzelkomponente nicht über 0,05 % wie n-Butyraldehyd, Dibutylether und gamma-Butyrolacton.

Durch Destillation in drei Kolonnen wird das Produkt gereinigt, wobei die erste Kolonne im Wesentlichen Wasser, Butanol und gamma-Butyrolacton über Sumpf ausschleust, die zweite Kolonne, die bei einem höheren Druck als die erste Kolonne betrieben wird, über Kopf ein Wasser/THF-Azeotrop abdestilliert, welches in die erste Kolonne rückgeführt wird, und über Sumpf wasserfreies THF ergibt, welches in einer dritten Kolonne im Wesentlichen von Butyraldehyd (Sumpfprodukt) befreit wird. Das resultierende THF hat eine Reinheit von > 99,9 % und kann als solches beispielsweise für die Herstellung von PolyTHF verwendet werden. Es enthält weniger als 1 Gew.-ppm N.

Die 50 ml Cu-Katalysator-Tabletten wurden unter Stickstoffschutzgas in 5 gleichen Fraktionen ausgebaut und auf ihren Schwefelgehalt hin untersucht. Gegenüber einem Schwefelgehalt von 0,01 Gew.-% im ungebrauchten Katalysator lagen die Schwefelgehalte in den ersten beiden Schichten bei 1,5 bzw. 0,3 Gew.-%, in der dritten Schicht bei 0,1 % und in der 4. Und 5. Schicht bei ca. 0,02 %. Unter den Reaktionsbedingen liegt die maximale Aufnahmekapazität des Schwefels demnach bei mindestens 1,5 Gew.-%.

### Hydrierungen zu 1,4-Butandiol

### Vergleichsbeispiel 3:

Das Bernsteinsäureanhydrid aus Beispiel 1 wurde als 20 Gew.-%ige, wässrige Lösung an einem Re/Pt/C-Katalysator analog Beispiel 1 der DE10009817 A1 hydriert (Zulauf 100 g/h, Temperatur 155°C, Druck 220bar, 20 Mol Wasserstoff/h, 120 ml Katalysator, Rohrreaktor, 2 cm Durchmesser, Rieselfahrweise. Anfänglich betrug die 1,4-Butandiolausbeute ca. 95 % bei 100% Umsatz (Rest Butanol, Propanol, THF und gamma-Butyrolacton). Bereits nach 100 h verringerte sich der Umsatz auf 98% und die Butandiolausbeute lag nur noch bei 90 %.

### Beispiel 4 (erfindungsgemäß):

Beispiel 3 wurde wiederholt, mit dem Unterschied, dass 50 g/h Bernsteinsäureanhydrid bei 125 °C über 100 ml des Katalysators (CuO (60 Gew.-%)/Al₂O₃ 3 mm Tabletten) aus Beispiel 2b bei 1,5 bar Überdruck und 5 Normliter Wasserstoff/h in Sumpffahrweise (Rohrreaktor, Ölbeheizt, 2 cm Durchmesser) geleitet wurden. Der Austrag wurde gesammelt, entsprechend Vergleichsbeispiel 3 in Wasser gelöst und wie dort beschrieben hydriert. Das Hydrierergebnis war auch nach 100 h so wie zu Beginn (98 % Butandiolausbeute, 100% Umsatz).

## Patentansprüche

1. Verfahren zur Herstellung von Tetrahydrofuran und/oder 1,4-Butandiol und/oder gamma-Butyrolacton, umfassend die Schritte
a) fermentative Herstellung von Bernsteinsäure,
b) Überführung der Bernsteinsäure aus Schritt a) unter Wasserabspaltung und Wasserabtrennung in Bernsteinsäureanhydrid,
c) Überführung des Bernsteinsäureanhydrids aus Schritt b) in die Gasphase,
d) Entfernen von schwefelhaltigen Verbindungen aus dem Bernsteinsäureanhydrid durch Leiten des gasförmigen Bernsteinsäureanhydrids aus Schritt c) über ein festes Schutzbett (guard bed), das die schwefelhaltigen Verbindungen absorbiert,
e) Hydrieren des gasförmigen Bernsteinsäureanhydrids aus Schritt d) in Gegenwart von freiem Wasserstoff an einem metallhaltigen Katalysator zu Tetrahydrofuran und/oder 1,4-Butandiol und/oder gamma-Butyrolacton.

2. Verfahren nach Anspruch 1, **dadurch gekennzeichnet, dass** in Schritt a) Bernsteinsäure fermentativ aus mindestens einer Kohlenstoffquelle hergestellt und nach Abtrennen der Biomasse aus der Fermentationsbrühe durch Ansäuern in die Säureform überführt wird.

3. Verfahren nach Anspruch 2, **dadurch gekennzeichnet, dass** die Bernsteinsäure aus Schritt a) ohne weitere Reinigungsschritte in Schritt b) überführt wird.

4. Verfahren nach einem der Ansprüche 1 bis 3, **dadurch gekennzeichnet, dass** die Wasserabspaltung und Wasserabtrennung in mindestens einer Verdampfungsvorrichtung bei einem Druck im Bereich von 0,05 bis 1 bar und einer Sumpftemperatur im Bereich von 100 bis 250°C erfolgt.

5. Verfahren nach einem der Ansprüche 1 bis 4, **dadurch gekennzeichnet, dass** Schritt c) in mindestens einer Destillationsvorrichtung bei einem Kopfdruck im Bereich von 0,02 bis 2 bar und einer Sumpftemperatur im Bereich von 100 bis 300°C unter Abtrennung von Hochsiedern über den Sumpf durchgeführt wird.

6. Verfahren nach Anspruch 5, **dadurch gekennzeichnet, dass** Schritt c) bei niedrigerem Druck als Schritt b) durchgeführt wird und in Schritt c) Wasser und gegebenenfalls Leichtsieder über Kopf abgetrennt werden und gasförmiges Bernsteinsäureanhydrid über einen Seitenabzug gewonnen wird.

7. Verfahren nach einem der Ansprüche 1 bis 3, **dadurch gekennzeichnet, dass** Schritt b) und Schritt c) zusammengefasst und in mindestens einer Destillationsvorrichtung bei einem Kopfdruck im Bereich von 0,02 bis 2 bar und einer Sumpftemperatur im Bereich von 100 bis 200°C unter Abtrennung von Hochsiedern über den Sumpf, Abtrennung von Wasser über Kopf und Gewinnung von gasförmigem Bernsteinsäureanhydrid über einen Seitenabzug durchgeführt werden.

8. Verfahren nach einem der Ansprüche 1 bis 7, **dadurch gekennzeichnet, dass** Schritt e) bei einem Druck im Bereich von 1 bis 65 bar und einer Temperatur im Bereich von 150 bis 350°C durchgeführt wird.

9. Verfahren nach einem der Ansprüche 1 bis 8, **dadurch gekennzeichnet, dass** im Hydrierkatalysator in Schritt e) das Hydriermetall ausgewählt ist aus Ru, Re, Co, Cu und Gemischen davon.

10. Verfahren nach einem der Ansprüche 1 bis 9, **dadurch gekennzeichnet, dass** der Hydrierkatalysator in Schritt e) ein Trägerkatalysator ist, in dem das Trägermaterial Kohlenstoff und/oder mindestens ein Oxid von B, Al, Si, Ti, Zr, La, Ce und/oder Cr enthält.

11. Verfahren nach einem der Ansprüche 1 bis 10, **dadurch gekennzeichnet, dass** das feste Schutzbett in Schritt d) ein Schwefel bindendes Metall, ausgewählt aus Ru, Re, Co, Cu und Gemischen davon, enthält, und vorzugsweise dieselben Inhaltsstoffe und/oder dieselbe Zusammensetzung wie der Hydrierkatalysator in Schritt e) aufweist.

12. Verfahren nach einem der Ansprüche 1 bis 11, weiterhin nach Schritt e) den folgenden Schritt f) umfassend
f) destillative Abtrennung von Wasser und Hochsiedern vom Tetrahydrofuran, 1,4-Butandiol und/oder gamma-Butyrolacton.

13. Verfahren nach Anspruch 12, **dadurch gekennzeichnet, dass** Schritt f) bei der Herstellung von Tetrahydrofuran in mindestens drei Destillationskolonnen durchgeführt wird, wobei
f1) in einer ersten Destillationskolonne im Sumpf Hochsieder abgetrennt werden und über Kopf ein Tetrahydrofuran/Wasser-Azeotrop gewonnen wird,
f2) das Tetrahydrofuran/Wasser-Azeotrop aus Schritt f1) in einer zweiten Kolonne, die bei höherem Druck als die erste Kolonne in Schritt f1) betrieben wird, aufgetrennt wird in ein Tetrahydrofuran/Wasser-Azeotrop, das über Kopf abgetrennt und vorzugsweise in Schritt f1) zurückgeführt wird, und Tetrahydrofuran, das über den Sumpf gewonnen wird,
f3) das in Schritt f2) über den Sumpf gewonnene Tetrahydrofuran in einer dritten Kolonne von Hochsiedern, die über den Sumpf ausgetragen werden, befreit wird.

## Claims

1. A process for preparing tetrahydrofuran and/or butane-1,4-diol and/or gamma-butyrolactone, comprising the steps of
a) fermentative preparation of succinic acid,
b) conversion of the succinic acid from step a) with elimination of water and removal of water to succinic anhydride,
c) conversion of the succinic anhydride from step b) to the gas phase,
d) removal of sulfur compounds from the succinic anhydride by passing the gaseous succinic anhydride from step c) through a fixed guard bed which absorbs the sulfur compounds,
e) hydrogenation of the gaseous succinic anhydride from step d) in the presence of free hydrogen over a metallic catalyst to give tetrahydrofuran and/or butane-1,4-diol and/or gamma-butyrolactone.

2. The process according to claim 1, wherein succinic acid is prepared in step a) by fermentation from at least one carbon source and, after the biomass has been removed from the fermentation broth, is converted to the acid form by acidification.

3. The process according to claim 2, wherein the succinic acid from step a) is transferred into step b) without any further purification steps.

4. The process according to any of claims 1 to 3, wherein the elimination of water and removal of water are effected in at least one evaporation apparatus at a pressure in the range from 0.05 to 1 bar and a bottom temperature in the range from 100 to 250°C.

5. The process according to any of claims 1 to 4, wherein step c) is conducted in at least one distillation apparatus at a top pressure in the range from 0.02 to 2 bar and a bottom temperature in the range from 100 to 300°C with removal of high boilers via the bottom.

6. The process according to claim 5, wherein step c) is conducted at a lower pressure than step b) and, in step c), water and any low boilers are removed overhead and gaseous succinic anhydride is obtained via a side draw.

7. The process according to any of claims 1 to 3, wherein step b) and step c) are combined and are conducted in at least one distillation apparatus at a top pressure in the range from 0.02 to 2 bar and a bottom temperature in the range from 100 to 200°C with removal of high boilers via the bottom, removal of water overhead and recovery of gaseous succinic anhydride via a side draw.

8. The process according to any of claims 1 to 7, wherein step e) is conducted at a pressure in the range from 1 to 65 bar and a temperature in the range from 150 to 350°C.

9. The process according to any of claims 1 to 8, wherein the hydrogenation metal in the hydrogenation catalyst in step e) is selected from Ru, Re, Co, Cu and mixtures thereof.

10. The process according to any of claims 1 to 9, wherein the hydrogenation catalyst in step e) is a supported catalyst in which the support material comprises carbon and/or at least one oxide of B, Al, Si, Ti, Zr, La, Ce and/or Cr.

11. The process according to any of claims 1 to 10, wherein the fixed guard bed in step d) comprises a sulfur-binding metal selected from Ru, Re, Co, Cu and mixtures thereof, and preferably has the same ingredients and/or the same composition as the hydrogenation catalyst in step e).

12. The process according to any of claims 1 to 11, further comprising, after step e), the following step f)
f) distillative separation of water and high boilers from the tetrahydrofuran, butane-1,4-diol and/or gamma-butyrolactone.

13. The process according to claim 12, wherein step f) is conducted in the preparation of tetrahydrofuran in at least three distillation columns, wherein
f1) in a first distillation column high boilers are removed in the bottoms and a tetrahydrofuran/water azeotrope is obtained overhead,
f2) the tetrahydrofuran/water azeotrope from step f1) is separated in a second column which is operated at a higher pressure than the first column in step f1) into a tetrahydrofuran/water azeotrope which is removed overhead and preferably recycled into step f1), and tetrahydrofuran which is obtained via the bottom,
f3) the tetrahydrofuran obtained via the bottom in step f2) is freed in a third column of high boilers which are discharged via the bottom.

## Revendications

1. Procédé pour la préparation de tétrahydrofuranne et/ou de 1,4-butanediol et/ou de gamma-butyrolactone, comprenant les étapes :
a) préparation par fermentation d'acide succinique,
b) transformation de l'acide succinique de l'étape a) avec dissociation d'eau et séparation d'eau en anhydride de l'acide succinique,
c) transfert de l'anhydride de l'acide succinique de l'étape b) dans la phase gazeuse,
d) élimination de composés soufrés de l'anhydride de l'acide succinique par passage de l'anhydride de l'acide succinique gazeux de l'étape c) sur un lit de protection solide (guard bed), qui absorbe les composés soufrés,
e) hydrogénation de l'anhydride de l'acide succinique gazeux de l'étape d) en présence d'hydrogène libre sur un catalyseur métallique en tétrahydrofuranne et/ou en 1,4-butanediol et/ou en gamma-butyrolactone.

2. Procédé selon la revendication 1, **caractérisé en ce que**, dans l'étape a), l'acide succinique est préparé par fermentation à partir d'au moins une source de carbone et transformé en forme acide par acidification après séparation de la biomasse du bouillon de fermentation.

3. Procédé selon la revendication 2, **caractérisé en ce que** l'acide succinique de l'étape a) est transféré sans autres étapes de purification dans l'étape b).

4. Procédé selon l'une quelconque des revendications 1 à 3, **caractérisé en ce que** la dissociation d'eau et la séparation d'eau a lieu dans au moins un dispositif d'évaporation à une pression dans la plage de 0,05 à 1 bar et à une température du fond dans la plage de 100 à 250°C.

5. Procédé selon l'une quelconque des revendications 1 à 4, **caractérisé en ce que** l'étape c) est réalisée dans au moins un dispositif de distillation à une pression de tête dans la plage de 0,02 à 2 bars et à une température du fond dans la plage de 100 à 300°C avec séparation des substances à point d'ébullition élevé via le fond.

6. Procédé selon la revendication 5, **caractérisé en ce que** l'étape c) est réalisée à une pression inférieure à celle de l'étape b) et, dans l'étape c), l'eau et le cas échéant les substances à bas point d'ébullition sont séparées via la tête et l'anhydride de l'acide succinique gazeux est obtenu via un soutirage latéral.

7. Procédé selon l'une quelconque des revendications 1 à 3, **caractérisé en ce que** l'étape b) et l'étape c) sont rassemblées et réalisées dans au moins un dispositif de distillation à une pression de tête dans la plage de 0,02 à 2 bars et à une température du fond dans la plage de 100 à 200°C avec séparation des substances à point d'ébullition élevé via le fond, séparation de l'eau via la tête et obtention d'anhydride de l'acide succinique gazeux via un soutirage latéral.

8. Procédé selon l'une quelconque des revendications 1 à 7, **caractérisé en ce que** l'étape e) est réalisée à une pression dans la plage de 1 à 65 bars et à une température dans la plage de 150 à 350°C.

9. Procédé selon l'une quelconque des revendications 1 à 8, **caractérisé en ce que** le métal d'hydrogénation dans le catalyseur d'hydrogénation dans l'étape e) est choisi parmi Ru, Re, Co, Cu et leurs mélanges.

10. Procédé selon l'une quelconque des revendications 1 à 9, **caractérisé en ce que** le catalyseur d'hydrogénation dans l'étape e) est un catalyseur supporté, dans lequel le matériau support contient du carbone et/ou au moins un oxyde de B, d'Al, de Si, de Ti, de Zr, de La, de Ce et/ou de Cr.

11. Procédé selon l'une quelconque des revendications 1 à 10, **caractérisé en ce que** le lit de protection solide dans l'étape d) contient un métal liant le soufre, choisi parmi Ru, Re, Co, Cu et leurs mélanges et présente de préférence les mêmes constituants et/ou la même composition que le catalyseur d'hydrogénation dans l'étape e).

12. Procédé selon l'une quelconque des revendications 1 à 11, comprenant en outre, après l'étape e), l'étape suivante :
f) séparation par distillation de l'eau et des substances à point d'ébullition élevé du tétrahydrofuranne, du 1,4-butanediol et/ou de la gamma-butyrolactone.

13. Procédé selon la revendication 12, **caractérisé en ce que**, lors de la préparation de tétrahydrofuranne, l'étape f) est réalisée dans au moins trois colonnes de distillation, où
f1) dans une première colonne de distillation, les substances à point d'ébullition élevé sont séparées dans le fond et un azéotrope tétrahydrofuranne/eau est obtenu via la tête,
f2) l'azéotrope tétrahydrofuranne/eau de l'étape f1) est séparé, dans une deuxième colonne, qui est exploitée à une température supérieure à celle de la première colonne dans l'étape f1), en un azéotrope tétrahydrofuranne/eau, qui est séparé via la tête et de préférence recyclé dans l'étape f1), et en tétrahydrofuranne, qui est obtenu via le fond,
f3) le tétrahydrofuranne obtenu via le fond dans l'étape f2) est libéré dans une troisième colonne des substances à point d'ébullition élevé, qui sont évacuées via le fond.
